# EUROPEAN PATENT APPLICATION

(11) **EP 4 299 560 A1**
(43) Date of publication of application: **03.01.2024**
(21) Application number: 22182535.9
(22) Date of filing: 01.07.2022
(51) Int. Cl.: C07C 315/02, C07C 315/04, C07C 317/46, C07C 317/48

(54) **METHOD FOR THE PRODUCTION OF ALPHA HYDROXY-ALKYLTHIO CARBOXYLIC ACIDS AND DERIVATIVES THEREOF**

(71) Applicant: AMINO GmbH, 38373 Frellstedt (DE)
(72) Inventor: Bischoff, Matthias, 38100 Braunschweig (DE)
(74) Representative: Gramm, Lins & Partner Patent- und Rechtsanwälte PartGmbB

(57) **Abstract**

In a first aspect, the present invention provides a method for producing a compound of general formula (I) starting from methionine with an intermediate product of methionine sulfoxide. The method according to the present invention allows to produce the desired compound of general formula (I), in particular, the compound calcium-2-hydroxy-4-methylthio butyric acid (Ca-MHA) with high yields and a purity not requiring any additional purification steps after synthesis. In particular, the present invention relates to a one pot synthesis method for producing MHA. Further, the alpha Hydroxy-alkylthio carboxylic acid obtained according to the present invention is provided.

## Description

In a first aspect, the present invention provides a method for producing a compound of general formula (I) starting from methionine with an intermediate product of methionine sulfoxide. The method according to the present invention allows to produce the desired compound of general formula (I), in particular, the compound calcium-2-hydroxy-4-methylthio butyric acid (Ca-MHA) with high yields and a purity not requiring any additional purification steps after synthesis. In particular, the present invention relates to a one pot synthesis method for producing MHA. Further, the alpha Hydroxy-alkylthio carboxylic acid obtained according to the present invention is provided.

### Prior Art

Methionine is an essential sulfur-containing amino acid which plays a major role as feed additive in livestock nutrition. Methionine increases the biological value of proteins in feed for livestock and leads to faster growth. In addition, analogues of methionine and other essential or non-essential amino acids are used in nutrition and supplements e. g. in nutrition therapy.

Namely, keto- and hydroxy analogues of amino acids are known for various application in complimentary therapy in various disease. For example, ketosteril is a known supplement in nutritional therapy in chronic kidney disease. Ketosteril allows the intake of essential amino acids while minimizing the amino nitrogen intake. Ketosteril is a composition composed of various keto- and hydroxy analogues, namely the calcium salts of alpha-Ketoisoleucine, alpha-Ketoleucine, alpha-Ketophenylalanine, alpha-Ketovaline and alpha-Hydroxymethionine as well as other amino acids.

The keto- and hydroxy analogues are converted to the corresponding amino acids in the body. Therefore, MHA has an equal commercial importance not only in livestock nutrition, but also, e. g. as part of ketosteril, in nutritional and pharmaceutical applications in men.

Synthesis of the alpha-hydroxy-alkylthio carboxylic acids, including MHA, are described in the art. The most widely used process for the production of MHA to date is based on the conjugate addition of methyl mercaptan to acrolein to form 3-(methylthio)propionaldehyde. The aldehyde is reacted with hydrocyanic acid to form the corresponding cyanohydrin. The cyano group is then reacted with sulfuric acid via the amide to form the carboxylic acid. The method is described e. g. in US 2,745,745 A, an WO 96/05173 A1. However, a main disadvantage of this widely used method is the use of the highly toxic cyanide.

Other processes are described in the art, e. g. a second process is described including the step of addition of sodium methylthiolate to α-hydroxy-γ-butyrolactone in polar aprotic solvents, like DMF or DMSO, at elevated temperature to obtain MHA, see e. g. EP 245231 A1 or EP 498765 A1 as well as WO 2008/022953 A1. However, the availability of the starting material is difficult. Further, the use of the strong bases in polar aprotic solvents at elevated temperatures poses a safety risk.

Another method for the synthesis of MHA from methionine by deazotisation using concentrated sulfuric acid and sodium nitrite is described by Stavrakov, G. et al., Eur. J. Med. Chem., 2013, 70, 372-379, however, MHA is obtained in very low yields. Recently, another method for the preparation of MHA is disclosed in CN 112645857 A, using this very method.

Hence, there is a need for methods improving the overall yields of the alpha-hydroxy-alkylthio carboxylic acids, in particular, MHA. In particular, it is desired to provide a cost effective method for producing the alpha-hydroxy-alkylthio carboxylic acids with high yields and with a purity not requiring further purification processes.

### Brief description of the present invention

The present invention provides a new process for the production of alpha-hydroxy-alkylthio carboxylic acids in particular MHA, at high yields, which is an efficient process that can be economically implemented on a large scale. In particular, the present invention provides a method for preparing the alpha-hydroxy-alkylthio carboxylic acids, like MHA, and its salt with the respective amino acid as a starting material being efficient and cost effective.

According to a first aspect of the present invention, a method for producing the alpha-hydroxy-alkylthio carboxylic acids of the compounds of general formula (I) is provided: 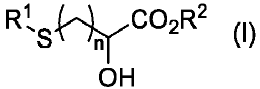 wherein R¹ is a C₁ - C₆ alkyl group, like a C₁ - C₄ alkyl group,
n is an integer of 1 to 4, and
R² is selected from H or a C₁ - C₄ alkyl group, characterized in, that a compound of the general formula (II) 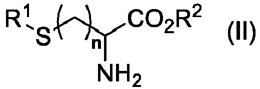 is reacted with an oxidizing agent to obtain a compound of the general formula (III) 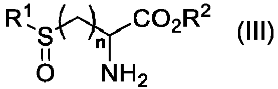 the compound of formula (III) is reacted with a reagent for converting the amino group into a hydroxy group to produce the compound of general formula (IV), 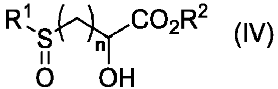 wherein the compound of structure (IV) is reacted with a reducing agent to obtain the compound of general formula (I), which may be optionally present in form of salts, e.g. according to formula (Ia) 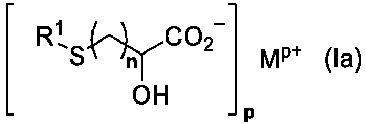 or solvates thereof.

In particular, the present invention provides a method for producing MHA, like the calcium salt of MHA, based on the method described above. The method may be conducted in a one pot reaction with high yields and with high purity of the MHA obtained of at least 95 %, like at least 98 %, thus, not requiring further process steps for purification of the MHA.

In another aspect, the compound of general formula (I) obtainable according to the method of the present invention is provided.

### Brief description of the figures

Figure 1 is a general scheme of the method according to the present invention.
Figure 2: HPLC chromatogram of D,L-methionine sulfoxide, see example 1
Figure 3: HPLC chromatogram of MHA, see examples 2, 3.
Figure 4: HPLC chromatogram of Ca-MHA, see example 4.

### Detailed description of the present invention

The present invention relates to a method for producing a compound of the general formula (I), 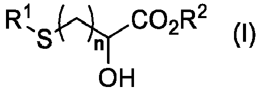 wherein R¹ is a C₁ - C₆ alkyl group, like a C₁ - C₄ alkyl group,
n is an integer of 1 to 4, and
R² is selected from H or a C₁ - C₄ alkyl group, characterized in, that a compound of the general formula (II) 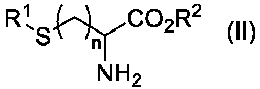 is reacted with an oxidizing agent to obtain a compound of the general formula (III) 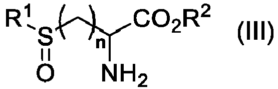 the compound of formula (III) is reacted with a reagent for converting the amino group into a hydroxy group to produce the compound of general formula (IV), 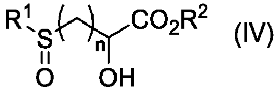 wherein the compound of structure (IV) is reacted with a reducing agent to obtain the compound of general formula (I), which may be optionally present in form of salts or solvates thereof.

As used herein, the term "reagent for converting the amino group into a hydroxy group" refers to reagents capable of producing nitrosyl cations for converting the amino group present in general formula (III) into a hydroxy group as described.

Depending on the starting material of general formula (II), the alpha-hydroxy-alkylthio carboxylic acids like the MHA, can be obtained as racemic mixture or enantiomerically pure D- or L-MHA.

As used herein, the term "C₁ to C₆ alkyl group" refers to all possible embodiments of C₁ to C₆ alkyl, including methyl, ethyl, n-propyl, 1-methylethyl, n-butyl, 1-methylpropyl, 2-methylpropyl, 1,1-demethylethyl, n-pentyl, 1-methyl-butyl, 2-methylbutyl, 2-methylbutyl, 2,2-dimethylpropyl, 1-ethylpropyl, 1,1-dimethylpropyl, 1,2-dimethylpropyl and hexyl, 1-methylpenthyl, 2-methylpenthyl, 3-methylpenthyl, 4-methylpenthyl, 1,1-dimethylbutyl, 1,2-dimethylbutyl, 1,3-dimethylbutyl, 2,2-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1-ethyl-1-1-methylpropyl, 1-ethyl-3-methylpropyl. Preferably, the C₁ to C₆ alkyl group is a C₁ to C₄ alkyl group.

The C₁ to C₄ alkyl group includes the embodiments of ethyl, methyl, n-propyl, 1-methylethyl, butyl, 1-methylpropyl, 2-methylpropyl, 1,1-dimethylethyl.

In a particularly preferred embodiment, R¹ is methyl.

As said, R¹ is an alkyl group, in particular, C₁ to C₄ alkyl group, like methyl.

Further, R² is selected from hydrogen or a C₁ to C₄ alkyl group, a C₁ to C₄ alkyl group as defined above. In a preferred embodiment, R² is hydrogen.

Further, n is an integer of 1 to 4. As used herein, the integer of 1 to 4 includes the embodiments of 1, 2, 3, 4. In a preferred embodiment, n is 2.

In an embodiment, the alpha-hydroxy-alkylthio carboxylic acids according to the present invention are present in form of the salt as shown in general formula (Ia)

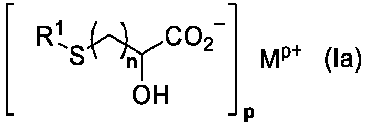

That is, according to the present invention p is an integer of 1, 2 or 3 depending on the cation M. P⁺ identifies the respective cation of the salt, whereby M is selected from an earth alkali metal, an alkali metal, Fe, Zn or NH₄⁺. In a preferred embodiment, M is an earth alkali metal, in particular, Ca.

That is, it is preferred that the process is a process for the production of MHA and the respective calcium salt.

The process according to the present invention comprises a first step wherein the compound of general formula (I), like methionine, is oxidized with a suitable oxidizing agent to e. g. methionine sulfoxide. For example, the synthesis is described in: Kupwade R. V., 2019, J. Chem. Rev., 1, 99-113.

Suitable oxidising agents are known to the skilled person, for example, the oxidizing agent is selected form the group of oxygen, potassium permanganate, nitric acid, cerium ammonium nitrate, sodium periodate, iodoxybenzoic acid, hydrogen peroxide, tert-butyl hydroxyperoxide, meta-chloroperbenzoic acid, sodium hypochlorite, potassium peroxomonosulfate, or mixtures thereof.

In an aspect of the present invention, the sulfoxide may be isolated, thus being the starting material for a second reaction, alternatively, in case of a one pot reaction the process is continued by reacting the compound of general formula (III) with a reagent for converting the amino group into a hydroxy group to produce the compound of general formula (IV). Typically, this is achieved by reacting the amine present in the compound according to general formula (III) with a nitrosyl cation. The skilled person is well aware of suitable reagents allowing the conversion of the amino group into a hydroxy group. For example, suitable reagents capable of producing nitrosyl cations are selected from nitrous acid, sodium nitrite, potassium nitrite, amyl nitrite, nitrosyl tetrafluoroborate or mixtures thereof.

The obtained compound of general formula (IV) is reacted further with a reducing agent to obtain the compound of general formula (I). The procedure is described e. g. in Madesclaire M., 1988, Tetrahedron, 44 (21), 6537-6580. The reducing agents may be selected from the group of hydrochloric acid, hydrobromic acid, sulfur containing compounds, including thiole, thioether, thiolic acid, and sulfur dioxide, phosphor containing compounds, like phosphine and phosphite, silicon containing compounds including silane, metal including zinc, titanium, molybdenum, rhenium or ruthenium in connection with a suitable oxygen acceptor. This reducing method is combined with a suitable oxygen acceptor known to a skilled person. For example, suitable oxygen acceptors include hydrogen, glycerol, boranes, phosphines, phosphites and silanes.

Finally, the compound of general formula (I) is converted into the salt according to general formula (Ia) 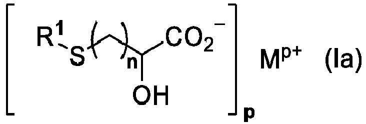 using suitable cation sources. E. g. for obtaining the calcium salt, the calcium is selected from calcium carbonate, calcium acetate, calcium chloride, calcium oxide and the like.

In a preferred embodiment, the process allows to obtain a compound of general formula (I) being calcium-2-hydroxy-4-methylthiobutyric acid (Ca-MHA).

The process according to the present invention may be conducted in several steps or may be conducted in a one pot reaction. Both possibilities are shown in the following examples.

The conditions for performing each of the reaction steps are known to the skilled person and become clear from the examples below.

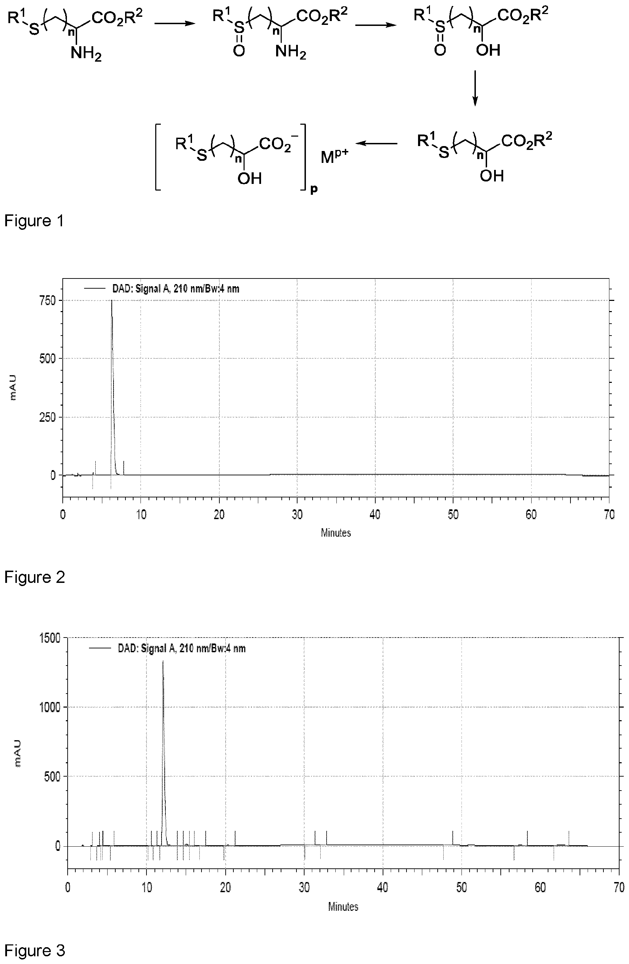

The general procedure of the new process for producing the alpha-hydroxy-alkylthio carboxylic acids, here the Ca-MHA is shown in the scheme, see figure 1. In short, D,L-methionine sulfoxide is obtained by reacting D,L-methionine with hydrogen peroxide in the presence of acetic acid or water at temperatures between 0°C and room temperature for one hour. The process is continued by adding sulfuric acid and an aqueous solution of sodium nitrite at a temperature below room temperature or at room temperature, continued by further stirring at room temperature or elevated temperatures.

The sulfoxide obtained is reacted further by adding sodium pyrosulfite and stirring at room temperature or under heating to obtain the alpha-hydroxy-alkylthio carboxylic acid, here MHA. If desired, the salt may be obtained by adding calcium chloride under basic conditions and heating to obtain Ca-MHA accordingly.

In a further aspect, the present invention relates to the alpha-hydroxy-alkylthio carboxylic acid obtainable by the process according to the present invention. Namely, the alpha-hydroxy-alkylthio carboxylic acid, in particular, the MHA, is obtained with high purity of above 95 %, in particular, above 98 %, like 99 %, for example 99.5 %. Thus, the product obtained according to the present invention can be used without any additional purification steps necessary to receive the required purification grade for the compound when used for nutritional purposes. In particular, the process allows to obtain material which has a high purity for immediate use in applications requiring specific approval, like in the nutritional field and pharmaceutical field.

Moreover, the process according to the present invention allows to produce the desired alpha-hydroxy-alkylthio carboxylic acid, in particular, the MHA components with higher yield and higher purity compared to the prior art. For example, the overall yield in prior art e.g. in CN112645857 A is about 23% with a purity of 97%.

The present invention will be described further by way of examples without limiting the same thereto:

### Analytical Methods:

Analytical measurements were performed on the Agilent 1260 Infinity II HPLC system. HPLC column Agilent Zorbax SB-Aq (250 × 4.6 mm), 5 µm; flow rate 1.1 ml/min, detection wavelength 210 nm; solvent A: KH₂PO₄, CH₃(CH₂)₇SO₃Na, H₃PO₄, H₂O, pH = 2; solvent B: solvent A / MeCN 1:1; gradient: 0 - 15 min 100% → 98% A, 15 - 35 min 98% → 80% A, 35 - 60 min 80% A, 60 - 62 min 80% → 100% A, 62 - 70 min 100% A.

### Examples:

### Example 1: Synthesis of D,L-methionine sulfoxide.

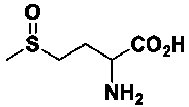

To a suspension of D,L-Methionine (50.0 g, 335 mmol) in AcOH (400 mL) at 0-10 °C, H₂O₂ (30% in H₂O, 34.2 mL, 1.0 eq.) was added dropwise, and stirred at 0-10 °C for 10 min. The cooling bath was removed and stirred for 30 min at 20-25 °C. MnO₂ (50 mg) was then added and stirred until the gas evolution ceased. The reaction mixture was filtered, washed with a little AcOH and the solvent was removed under reduced pressure at 45 °C. The residue was dissolved in H₂O (50 mL) and acetone (500 mL) was added. The precipitated solid was filtered off, washed with acetone (100 mL) and dried under reduced pressure at 50-60 °C for 14-16 h. A colorless solid (52.0 g, 315 mmol, 94%) was obtained. (Helv. Chim. Acta 1961, 8, 61-78).

HPLC: t_{R} = 6.28 min, purity >99.0%, see figure 2

### Example 2: Synthesis of 2-hydroxy-4-methylthiobutyric acid starting from D,L-methionine sulfoxide.

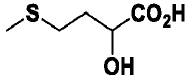

D,L-methionine sulfoxide (20.0 g, 121 mmol) was dissolved in H₂O (143 mL) and conc. H₂SO₄ (6.71 mL, 121 mmol, 1.0 eq.), and a solution of NaNO₂ (8.35 g, 121 mmol, 1.0 eq.) in H₂O (50 mL) was added over a period of 15-30 min followed by stirring for 2-6 h.

To the reaction solution was added Na₂S₂O₅ (57.5 g, 303 mmol, 2.5 eq.), it was heated to 50-70 °C and stirred for 1-3 h. The reaction solution was allowed to reach room temperature, extracted with EtOAc (2 × 200 mL), dried over Na₂SO₄, filtered, and the solvent was removed under reduced pressure at 45 °C. A yellow-colored oil (8.80 g, 58.6 mmol, 48%) was obtained.

HPLC: t_{R} = 12.4 min, purity >97.0%, see figure 3

### Example 3: Synthesis of 2-hydroxy-4-methylthiobutyric acid starting from D,L-methionine.

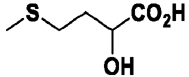

To a suspension of D,L-methionine (20.0 g, 134 mmol) in H₂O (140 mL) at 0-10 °C, H₂O₂ (30% in H₂O, 13.7 mL, 1.0 eq.) was added dropwise, and stirred for 10 min at 0-10 °C. The cooling bath was removed and stirred for 30 min at 20-25 °C. MnO₂ (50 mg) was then added and stirred until the gas evolution ceased.

Conc. H₂SO₄ (7.29 mL, 134 mmol, 1.0 eq.) was added to the reaction solution, and a solution of NaNO₂ (11. g, 161 mmol, 1.2 eq.) in H₂O (20 mL) was added over a period of 15-30 min, followed by stirring for 1-6 h.

Na₂S₂O₅ (50.9 g, 268 mmol, 2.0 eq.) was added to the reaction solution, it was heated to 50-70 °C and stirred for 1-3 h. The reaction solution was then allowed to reach room temperature, extracted with EtOAc (2 × 200 mL), dried over Na₂SO₄, filtered, and the solvent was removed under reduced pressure at 45 °C. A yellow-colored oil (10.4 g, 34.0 mmol, 52%) was obtained.

HPLC: t_{R} = 12.4 min, purity >97.0%, see figure 3.

### Example 4: Synthesis of calcium 2-hydroxy-4-methylthiobutyric acid.

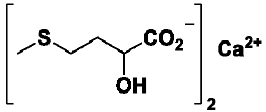

2-Hydroxy-4-methylthiobutyric acid (10.0 g, 66.6 mmol) was dissolved in H₂O (40 mL), and a pH of 6-7 was adjusted by adding 30% aqueous NaOH solution. The solution was heated to 75-85 °C, CaCl₂ (3.69 g, 33.3 mmol, 0.5 eq.) was added, and stirring was performed for 30-60 min at 75-85 °C. After cooling to 20-25 °C, the solid was filtered off, washed with H₂O (10 mL), and dried under reduced pressure at 50-60 °C for 14-16 h. A colorless solid (7.31 g, 21.6 mmol, 65%) was obtained.

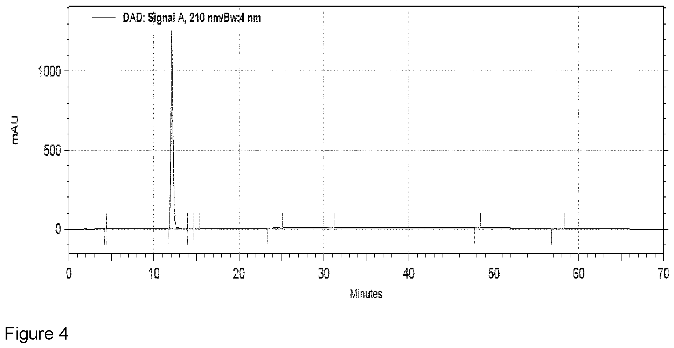

HPLC: t_{R} = 12.4 min, purity >99.5%, see figure 4.

## Claims

1. Method for producing a compound of the general formula (I), 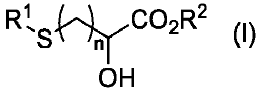 wherein R¹ is a C₁ - C₆ alkyl group, like a C₁ - C₄ alkyl group,
n is an integer of 1 to 4, and
R² is selected from H or a C₁ - C₄ alkyl group, **characterized in, that** a compound of the general formula (II) 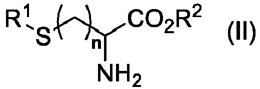 is reacted with an oxidizing agent to obtain a compound of the general formula (III) 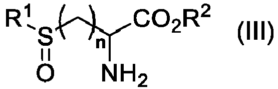 the compound of formula (III) is reacted with a reagent for converting the amino group into a hydroxy group to produce the compound of general formula (IV), 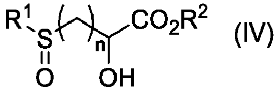 wherein the compound of structure (IV) is reacted with a reducing agent to obtain the compound of general formula (I), which may be optionally present in form of salts or solvates thereof.

2. The method according to claim 1, wherein the compound of formula (I) is in form of a salt of general formula (Ia), 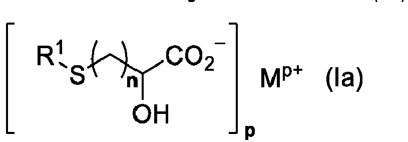 wherein p is an integer of 1, 2 or 3 and M is selected from an earth alkali metal, an alkali metal, Fe, Zn or NH₄⁺.

3. The method for producing a compound of general formula (I), wherein R¹ is a methyl group and/or R² is a hydrogen.

4. The method for producing a compound of general formula (I) according to any one of the preceding claims, wherein n is 2.

5. The method according to any one of the preceding claims, wherein the compound of the general formula (I) is obtained as racemic mixture.

6. The method for producing a compound of general formula (I) according to any one of claims 2 to 5, wherein the salt according to general formula (Ia) is a salt, wherein M is an earth alkali metal, in particular, Ca.

7. The method for producing a compound of general formula (I) according to any one of the preceding claims, wherein the oxidizing agent is selected from the group of oxygen, potassium permanganate, nitric acid, cerium ammonium nitrate, sodium periodate, iodoxybenzoic acid, hydrogen peroxide, tert-butyl hydroxyperoxide, meta-chloroperbenzoic acid, sodium hypochlorite, potassium peroxomonosulfate, or mixtures thereof.

8. The method for producing a compound of general formula (I) according to any one of the preceding claims, wherein the amine present in general formula (III) is reacted with reagents capable of producing nitrosyl cations, in particular, a reagent selected from nitrous acid, sodium nitrite, potassium nitrite, amyl nitrite, nitrosyltetrafluoroborate or mixtures thereof.

9. The method for producing a compound of general formula (I) according to any one of the preceding claims, **characterized in, that** the reducing agent is selected from hydrochloric acid, hydrobromic acid, sulfur containing compounds, including thiole, thioether, thiolic acid, and sulphur dioxide, phosphor containing compounds, like phosphine and phosphite, silicon containing compounds including silane, metal including zinc, titanium, molybdenum, rhenium or ruthenium in connection with a suitable oxygen acceptor.

10. The method for producing a compound of general formula (I) according to any one of the preceding claims, **characterized in, that** the formation of the salt, in particular, the calcium salt is at increased temperature under basic conditions.

11. The method for producing a compound of general formula (I) according to any one of the preceding claims, **characterized in, that** the production of the compound of general formula (I) from the compound of general formula (II) is in a one pot reaction.

12. The method for producing a compound of general formula (I) according to any one of the preceding claims, **characterized in, that** the compound of general formula (I) is calcium 2-hydroxy-4-methylthiobutyric acid.

13. The method for producing a compound of general formula (I) according to any one of the preceding claims, **characterized in, that** after synthesis without further purification the purity is at least 95 %, like at least 98 %.

14. Compound of general formula (I) obtainable according to a method according to any one of the claims 1 to 13.
